# EUROPÄISCHE PATENTSCHRIFT

(11) **EP 2 906 708 B1**
(45) Veröffentlichungstag und Bekanntmachung des Hinweises auf die Patenterteilung: **14.12.2016**
(21) Anmeldenummer: 13770914.3
(22) Anmeldetag: 01.10.2013
(51) Int. Cl.: C12P 7/64

(54) **DREISTUFIGES VERFAHREN ZUR ENZYMATISCHEN FETTSÄUREESTERSYNTHESE**
THREE-STAGE PROCESS FOR THE ENZYMATIC SYNTHESIS OF FATTY ACID ESTERS
PROCÉDÉ DE SYNTHÈSE ENZYMATIQUE D'ESTERS D'ACIDES GRAS À TROIS ÉTAPES

(30) Priorität: 10.10.2012 EP 12187899
(43) Veröffentlichungstag der Anmeldung: 19.08.2015
(73) Patentinhaber: BASF SE, 67056 Ludwigshafen am Rhein (DE)
(72) Erfinder: KEMPERS, Peter, 41189 Mönchengladbach (DE); SCHÖRKEN, Ulrich, 40591 Düsseldorf (DE); KAWA, Rolf, 40789 Monheim (DE); SCHWARZER, Jörg, 40723 Hilden (DE); WOLF, Thomas, 42781 Haan (DE)
(74) Vertreter: Blumberg vel Spalve, Jutta
(86) Internationale Anmeldenummer: PCT/EP2013/070408
(87) Internationale Veröffentlichungsnummer: WO 2014/056756

(56) Entgegenhaltungen:
- WO-A2-2007/017167
- US-A- 4 826 767
- HILLS, G.: "Industrial use of lipases to produce fatty acid esters", EUROPEAN JOURNAL OF LIPID SCIENCE AND TECHNOLOGY, Bd. 105, Nr. 10, 29. September 2003 (2003-09-29), Seiten 601-607, XP002695870, in der Anmeldung erwähnt

## Beschreibung

Die vorliegende Erfindung umfasst ein Verfahren zur Herstellung von Estern aus Fettalkoholen und Fettsäuren, bei dem der überwiegende Teil des während der Synthese entstandenen Wassers in flüssiger Form abgeführt wird.

Die enzymatische Synthese von Estern aus Fettalkoholen und Fettsäuren ist bekannt. Die enzymatische Synthese ist ein umweltschonendes Verfahren, wobei die Synthese unter vollständiger Verdampfung des gebildeten Reaktionswassers erfolgt.

Die industrielle Anwendung von Lipasen zur Herstellung von Fettsäureestern ist beispielsweise von Geoffrey Hills in Eur. J. Lipid Sci. Technol. 105, 2003, Seiten 601-607 beschrieben worden. Die hergestellten Fettsäureester, insbesondere Myristylmyristat, wird in einem Festbettverfahren hergestellt, wobei die Edukte mehrmals durch die Säule gepumpt werden und das entstandene Reaktionswasser unter Vakuum abgedampft wird.

Die Patentschrift US 4 826 767 A (HANSEN, T.T., 2. Mai 1989) offenbart ein Verfahren zur Herstellung von Estern aus Fettalkoholen und Fettsäuren umfassend den Schritt:
i) Umsetzen von Fettalkoholen der Formel R¹-OH, worin R¹ eine aliphatische lineare oder verzweigte Kohlenwasserstoffgruppe mit 6 bis 22 Kohlenstoffatomen bedeutet, und Fettsäuren der Formel R²-COOH, worin R² eine aliphatische lineare oder verzweigte Kohlenwasserstoffgruppe mit 6 bis 22 Kohlenstoffatomen bedeutet, in Gegenwart von Lipase unter Vakuum bei einer Temperatur von 60 bis 80 °C.

Die Patentschrift WO 2007/017167 A2 (COGNIS IP MANAGEMENT GMBH, 15. Februar 2007) offenbart ein Verfahren zur Herstellung von Estern aus Alkoholen und Fettsäuren umfassend die Schritte:
i) Umsetzen von Alkoholen der Formel R¹-OH, worin R¹ eine aliphatische lineare oder verzweigte Kohlenwasserstoffgruppe mit 1 bis 4 Kohlenstoffatomen bedeutet, und Fettsäuren der Formel R²-COOH, worin R² eine aliphatische lineare oder verzweigte Kohlenwasserstoffgruppe mit 6 bis 22 Kohlenstoffatomen bedeutet, in Gegenwart von Lipase bei einer Temperatur im Bereich von 20 bis 70 °C, bis das Reaktionsgleichgewicht erreicht ist,
ii) Entfernen des bei der Umsetzung gebildeten Wassers und des nicht umgesetzten Alkohols,
iii) erneutes Zugeben von Alkohol und
iv) Vervollständigen der Umsetzung in Gegenwart eines chemischen Katalysators unter Vakuum bei einer Temperatur von 150 bis 250 °C.

Fettsäureester werden in zunehmendem Maße in kosmetischen Hautpflegeprodukten als Ölkörper bzw. Emollients eingesetzt. Um die hohen Anforderungen des Marktes bezüglich sensorischer Eigenschaften und optimaler dermatologischer Verträglichkeit zu erfüllen, werden kontinuierlich neue Ölkörper und Ölkörpergemische entwickelt und getestet. Dazu gehören auch innovative Technologien zur Herstellung dieser Fettsäureester.

Wie bereits ausgeführt, entsteht bei der Estersynthese aus Fettalkoholen und Fettsäuren Wasser, das abgeführt werden muss, um das Reaktionsgleichgewicht in Richtung vollständiger Synthese zu verschieben. Bei der enzymatisch katalysierten Herstellung von Fettsäureestern muss bei relativ niedrigen Temperaturen gearbeitet werden, um die Enzymstabilität optimal zu halten. Demzufolge wird ein sehr starkes Vakuum benötigt, um das Wasser zu verdampfen. Diese Verdampfung ist sehr energieintensiv.

Es hat sich außerdem gezeigt, dass sich Probleme beim Upscaling in Batch-Reaktoren ergeben. In typischen Produktionsreaktoren mit 10-100m³ erfolgt die Verdampfung des Wassers durch den Druckabfall, bedingt durch die Füllhöhe, nur in den oberen Schichten. Es hat sich weiterhin herausgestellt, dass die Reaktion bis zu einem Umsatz von 80-90% sehr schnell ist und typischerweise in weniger als fünf Stunden abläuft. Das hat allerdings zur Folge, dass die entstehende Menge an Wasser in einem Produktionsreaktor während dieser Zeit technisch nicht oder nur mit einem sehr großen apparativen Aufwand entfernt werden kann. Des weiteren ist die benötigte Energiezufuhr, die für die Verdampfung des Reaktionswassers benötigt wird, aufgrund der niedrigen Innentemperatur im Reaktor nur schwer in das System einzubringen.

Ein weiteres Problem ist darin zu sehen, dass der Enzymkatalysator bei hohen Temperaturen nur sehr gering stabil ist bzw. inaktiv wird. Es ist bekannt, dass Enzyme sehr temperaturempfindlich sind. Somit kann die Estersynthese nicht bei Temperaturen von größer als 60-80°C stattfinden. Es hat sich insbesondere herausgestellt, dass die Langzeitstabilität der Enzyme in Anwesenheit von kurzkettigen Alkoholen und kurzkettigen Fettsäuren (C8/C10) bereits bei niedrigeren Temperaturen, etwa ab 40°C, sinkt, so dass die Reaktion optimaler Weise bei Temperaturen um 40°C durchgeführt werden sollte.

Ausgehend von diesen Problemen bzw. Nachteilen der enzymatischen Estersynthese des Standes der Technik ist die Aufgabe der vorliegenden Erfindung darin zu sehen, eine Technologie zu schaffen, mit der es möglich ist, Ester aus Fettalkoholen und Fettsäuren herzustellen, wobei ein geringerer Gesamtenergieverbrauch gewährleistet ist, keine Probleme beim Upscaling im großen Batch-Reaktoren auftreten und eine verbesserte Enzymstabilität gewährleistet wird.

Diese Aufgabe ist mit dem Verfahren gemäß Patentanspruch 1 gelöst worden.

Die vorliegende Erfindung betrifft ein Verfahren zur Herstellung von Estern aus Fettalkoholen und Fettsäuren, das die Schritte umfasst:
Umsetzen von Fettalkoholen der Formel R¹-OH, worin R¹ eine aliphatische lineare oder verzweigte Kohlenwasserstoffe mit 6 bis 22 Kohlenstoffatomen bedeutet, und Fettsäuren der Formel R²-COOH, worin R² eine aliphatische lineare oder verzweigte Kohlenwasserstoffgruppe mit 6 bis 22 Kohlenstoffatomen bedeutet, in Gegenwart von Lipase bei einer Temperatur im Bereich von 30 bis 50 °C, bis das Reaktionsgleichgewicht erreicht ist;
Entfernen des bei der Umsetzung gebildeten Wassers und
Vervollständigen der Umsetzung unter Vakuum bei einer Temperatur von 50 bis 80 °C.

Die Unteransprüche definieren bevorzugte Ausführungsformen des erfindungsgemäßen Verfahrens.

Das erfindungsgemäße Verfahren zur Herstellung von Estern aus Fettalkoholen und Fettsäuren stellt eine probate Lösung für die Probleme bzw. Nachteile dar, die sich bei den entsprechenden Verfahren aus dem Stand der Technik herauskristallisiert haben. Das vorliegende Verfahren ist ein dreistufiger enzymatischer Prozess, bei dem zuerst ohne Vakuum bei einer niedrigen Temperatur bis ins Reaktionsgleichgewicht synthetisiert wird. In einem zweiten Schritt wird das Reaktionswasser abgeführt. In der nächsten, dritten Reaktionsstufe wird der Fettsäureester bei höherer Temperatur als bei Stufe 1 unter Vakuum bis zum Vollumsatz synthetisiert.

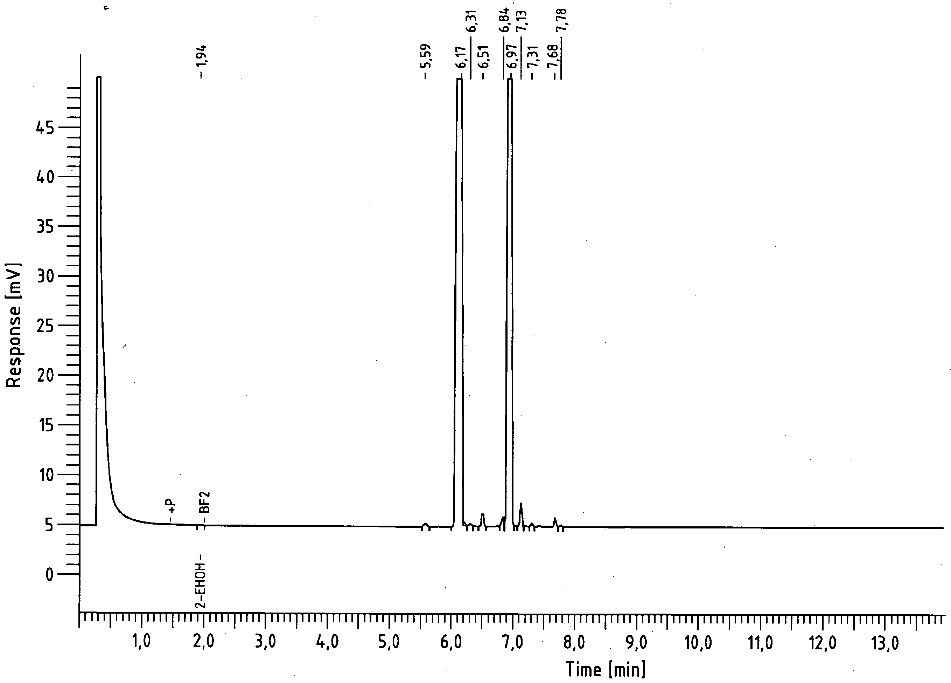

Die Figur 1 zeigt das GC-Chromatogramm eines erfindungsgemäß hergestellten Esters nach destillativer Aufreinigung.

In einer bevorzugten Ausführungsform des erfindungsgemäßen Verfahrens wird bei der Vervollständigung der Umsetzung, d. h. also bis zum Vollumsatz, in einer Stripgasatmosphäre synthetisiert. Als Stripgas können alle üblichen Stripgase verwendet werden. Beispiele für geeignete Stripgase sind Stickstoff, Luft, Kohlendioxid und Argon, wobei bevorzugt Stickstoff als Stripgas verwendet wird.

Ein wesentliches Charakteristikum des erfindungsgemäßen Verfahrens besteht darin, dass in der ersten Stufe zuerst ohne Vakuum, bis das Reaktionsgleichgewicht erreicht ist, gearbeitet wird. Erst nach Entfernen des bei der Umsetzung gebildeten Wassers, zum Beispiel durch Dekantieren, wird erfindungsgemäß die Umsetzung erneut gestartet, wobei dann ein Vakuum angelegt wird. Bei dieser Vervollständigung der Umsetzung wird ein Druck von 100 bis 10.000 Pa angewendet. In einer bevorzugten Ausführungsform des erfindungsgemäßen Verfahrens wird bei der Vervollständigung ein Druck von 1.500 bis 4.500 Pa angewendet.

Die Umsetzung, die anfangs ohne Vakuum durchgeführt wird, wird bei relativ niedrigen Temperaturen durchgeführt. Bevorzugt wird ein Temperaturbereich von 35 bis 45°C gewählt.

Bei der Vervollständigung der Umsetzung wendet man idealerweise einen höheren Temperaturbereich von 55 bis 65° C an.

Das erfindungsgemäße Verfahren erfolgt in Gegenwart einer Lipase. Eine bevorzugte Lipase ist die Lipase B, beispielsweise aus Candida antarctica. Die Lipase wird bevorzugter Weise in immobilisierter Form eingesetzt. Ein bevorzugtes Verfahren zur Immobilisierung ist beispielsweise die Adsorption an einem Trägermaterial. Beispiele für Trägermaterialien sind Accurel MP 1000 und Lewatit VPOC 1600. In der Praxis hat es sich als vorteilhaft erwiesen, wenn in der Synthese die immobilisierte Lipase in einer Menge von 0,5 bis 5,0 Gew.-% eingesetzt wird. Ein besonderer Vorteil des auf einem Träger immobilisierten Enzymkatalysators besteht auch darin, dass dieser wahrend des Entfernens des bei der Umsetzung gebildeten Wassers ohne weiteres über einem Sieb oder Filter zurückgehalten werden kann.

Das erfindungsgemäße Verfahren kann beispielsweise wie folgt durchgeführt werden:

In der ersten Stufe der enzymatischen Synthese wird im Batch-Reaktor mechanisch bewegt, z.B. gerührt, bis das Reaktionsgleichgewicht erreicht ist. In dieser Stufe wird mindestens 80% Umsatz Esterprodukt erreicht.

In der Zwischenstufe des Entfernens des bei der Umsetzung gebildeten Wassers wird die mechanische Bewegung des Ansatzes abgestellt, wonach man dann das Wasser bevorzugt für 0,5 bis 2 Sunden absitzen lässt. Das Wasser wird idealerweise über einen Bodenauslass abgelassen, wobei das Lipase immobilisat zurückgehalten wird, beispielsweise über ein Sieb im Reaktor oder Filtereinheit.

Die letzte Stufe der Vervollständigung der Umsetzung unter Vakuum führt man im gleichen Batch-Reaktor bis zu einem Umsatz von mindestens 95 %, bevorzugt größer als 95 % Umsatz Esterprodukt durch. Idealerweise kann die Lipase aus der ersten Stufe wieder zum Einsatz kommen, so dass ökonomisch gesehen die immobilisierte Lipase in der Gesamtsynthese wiederverwendet werden kann.

Die bei der Estersynthese verwendeten Ausgangsprodukte Fettalkohole und Fettsäuren können technische Fettalkohole oder Fettsäuren sein. Bevorzugt sind es allerdings Fettalkohole und Fettsäuren, die aus nachwachsenden Rohstoffen gewonnen werden.

Unter Fettalkoholen sind primäre aliphatische Alkohole der Formel zu verstehen,

R¹-OH

in der R¹ für einen aliphatischen, linearen oder verzweigten Kohlenwasserstoffrest mit 6 bis 22 Kohlenstoffatomen steht. Es sind ebenfalls ungesättigte Kohlenwasserstoffreste mit mindestens einer Doppelbindung eingeschlossen. Typische Beispiele sind Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleyl-alkohol, Behenylalkohol, Erucylalkohol und Brassidylalkohol sowie deren Mischungen. Bevorzugt sind Fettalkohole mit 8 bis 18 Kohlenstoffatomen.

Unter Fettsäuren sind aliphatische Carbonsäuren der Formel zu verstehen,

R²COOH

in der R² für einen aliphatischen, linearen oder verzweigten Kohlenwasserstoffrest mit 6 bis 22 Kohlenstoffatomen steht. Es sind ebenfalls ungesättigte Kohlenwasserstoffreste mit mindestens einer Doppelbindung eingeschlossen. Typische Beispiele sind Capronsäure, Caprylsäure, 2-Ethylhexansäure, Caprinsäure, Laurinsäure, Isotridecansäure, Myristinsäure, Palmitinsäure, Palm-oleinsäure, Stearinsäure, Isostearinsäure, Ölsäure, Elaidinsäure, Petroselinsäure, Linolsäure, Linolen-säure, Elaeostearinsäure, Arachinsäure, Gadoleinsäure, Behensäure und Erucasäüre sowie deren Mischungen. Bevorzugt sind technische Fettsäuren mit 8 bis 18 Kohlenstoffatomen.

Das erfindungsgemäße Verfahren zur Herstellung der Ester erfolgt bevorzugt im Eintopfverfahren. Ein bevorzugter Reaktor hierfür ist der Batch-Reaktor oder ein Batch-Reaktor mit Festbettschlaufe.

Zur Aufbereitung der erfindungsgemäß hergestellten Fettsäureester können weitere Aufreinigungs- und Konservierungsschritte durchgeführt werden. Dazu gehören beispielsweise eine Vorlaufdestillation zur Entfernung von nicht reagierten Edukten. So kann auch eine Produktdestillation zur farblichen Verbesserung erfolgen. Es hat sich herausgestellt, dass auch eine Desodorierung zur geruchlichen Optimierung erfolgreich durchgeführt werden kann. So kann auch die Zugabe von Antioxidantien (wie beispielsweise Tocopherol) zur Stabilisierung des Produkts ggf. erwünscht sein.

Das erfindungsgemäße Verfahren hat sehr viele Vorteile gegenüber den bekannten Verfahren zur Herstellung von Fettsäureestern. Ein wesentlicher Fortschritt besteht darin, dass ein außerordentlich geringer Gesamt-Energieverbrauch zu verzeichnen ist, da weniger Wasser verdampft wird im Gegensatz zu den Verfahren des Standes der Technik, bei denen die gesamte Reaktion unter Vakuum durchgeführt wird.
Im Zwischenschritt der zweiten Stufe, d. h. bei der Entfernung des Wassers, kann dieses vorteilhafterweise über einen Bodenauslass abgetrennt werden, da sich das Wasser sehr schnell von den hydrophoben Estern trennt. In großen Batch-Reaktoren lassen sich dadurch über dieses Verfahren kürzere Reaktionszeiten als bei kompletter Verdampfung des Reaktionswassers erreichen. Der Umsatz ins Reaktionsgleichgewicht ist praktisch unabhängig von der Batchgröße, er wird hauptsächlich durch die Vermischung beeinflusst.

Ein weiterer Vorteil ist darin zu sehen, dass eine außerordentlich verbesserte Enzymstabilität erreicht wird. Der Grund ist darin zu sehen, dass im ersten Verfahrensschritt bei deutlich niedriger Temperatur als in der dritten Stufe gearbeitet wird. Des weiteren ist in der dritten Stufe die Konzentration der desaktivierenden Komponenten, wie beispielsweise Ausgangsstoffe, deutlich niedriger.

Schließlich ist ein sehr großer Endumsatz zu verzeichnen. Das ist darauf zurückzuführen, dass bei erhöhter Temperatur in der dritten Stufe gearbeitet ist. Somit ist ein Umsatz bis größer als 99 % Produkt möglich. Der zusätzliche Einsatz eines Stripgases fördert weiterhin zur Steigerung des Endumsatzes.

Die folgenden Beispiele dienen zur Erläuterung des erfindungsgemäßen Verfahrens.

### Beispiele

### Beispiel 1: Langzeitstabilität des Enzyms im 3-Stufenverfahren (Labormaßstab)

Lewatit VPOC 1600 (Lanxess) und kommerziell erhältliche Lipase B aus *Candida antarctica* (Lipozym CalB L von Novozymes) wurden im Mengenverhältnis 1:1 über Nacht in der 20 fachen Menge Wasser bei Raumtemperatur gerührt. Das immobilisierte Enzym wurde abfiltriert und direkt zur Synthese eingesetzt.

In einen 1 l Reaktor mit Flügelrührer wurden 650 g einer equimolaren Mischung aus Oktanol und Fettsäure (technische Mischung aus Oktansäure und Dekansäure) mit 2 Gew% Enzymimmobilisat zur Reaktion gebracht. Die Mischung wurde für 4 h bei 45 °C unter Normaldruck gerührt. Nach 4 h wurde der Rührer gestoppt und eine Probe zur Säurezahlbestimmung entnommen. Nach 30 Min wurde die Wasserphase aus dem Reaktor abgelassen und die Reaktion wurde erneut gestartet. Die Temperatur wurde auf 60 °C erhöht, ein Ölpumpenvakuum von 20 mbar angelegt und die Reaktionsmischung wurde mit Stickstoff begast. Der Ansatz wurde für weitere 18 h bei konstanten Bedingungen gerührt. Nach dieser Reaktionszeit wurde der Ansatz abgelassen, der Umsatz über Säurezahlmessung bestimmt und es wurde mit dem gleichen Enzym ein neuer Ansatz unter gleichen Bedingungen gestartet. Insgesamt wurden 10 Ansätze mit dem gleichen Enzym durchgeführt. Die Startsäurezahl der Fettalkohol / Fettsäure Mischung lag bei 210.

| Ansatz | Säurezahl 4 h | Umsatz 4 h | Säurezahl 22 h | Umsatz 22 h |
|---|---|---|---|---|
| 1 | 26,8 | 87,2 % | 0,7 | 99,7 % |
| 2 | 25,9 | 87,7 % | 0,6 | 99,7 % |
| 3 | 25,8 | 87,7% | 3 | 98,6 % |
| 4 | 25,8 | 87,7 % | 0,7 | 99,7 % |
| 5 | 26,5 | 87,4 % | 4,8 | 97,7 % |
| 6 | 26,9 | 87,2 % | 0,9 | 99,6 % |
| 7 | 26,3 | 87,5 % | 5,1 | 97,6 % |
| 8 | 28,3 | 86,5 % | 2 | 99 % |
| 9 | 25,9 | 87,7 % | 1,6 | 99,2 % |
| 10 | 26,7 | 87,3, % | 1,8 | 99,1 % |

Nach 4 h Reaktionszeit erreichte die Reaktion über 10 Zyklen annähernd den Gleichgewichtszustand von knapp unter 90 % Umsatz. Nach 22 h Gesamtreaktionszeit unter Wasserentfernung durch Vakuum wurde in allen 10 Ansätzen ein Umsatz von > 97 % erreicht. Über 10 Ansätze wurde keine signifikante Abnahme der Enzymaktivität zu beobachten.

### Beispiel 2: Synthese nach 3-Stufenverfahren in einem 10 m³ Reaktor

Der 10 m³ Reaktor war ausgerüstet mit einem Flügelrührer, einer Doppelmantelbeheizung, einer Stickstoffbegasung am Reaktorboden und einer Strahler-Vakuumanlage. Zusätzlich waren im Bodenauslass Spaltensiebe zur Katalysatorrückhaltung installiert. Enzymimmobilisation und Reaktion wurden im gleichen Reaktor durchgeführt.

140 kg Accurel MP 1000 und 1500 kg Ethanol wurden in den Reaktor vorgelegt und für 0,5 h gerührt, danach wurde das Ethanol durch den Bodenauslass abgelassen. 3000 kg Wasser wurden in den Reaktor gegeben und unter Rühren wurden 150 kg Lipozym CalB L zugegeben. Die Immobilisation wurde für 16 h bei 25 °C durchgeführt. Die wässrige Lösung wurde aus dem Reaktor abgelassen und das zurückgehaltene Immobilisat wurde einmal mit Wasser gewaschen.

In den Reaktor wurden zum gesamten Enzymimmobilisat 6550 kg einer Mischung aus Oktanol und Fettsäure (technische Mischung aus Oktansäure und Dekansäure) gegeben. Die Mischung wurde für 4 h bei 45 °C und Normaldruck gerührt. Nach 4 h wurde der Rührer gestoppt und eine Probe zur Säurezahlbestimmung entnommen. Nach 30 min wurde die Wasserphase (etwa 350 kg) aus dem Reaktor abgelassen und die Reaktion wurde erneut gestartet. Die Temperatur wurde auf 60 °C erhöht, ein Strahlervakuum von etwa 30 mbar angelegt und die Reaktionsmischung wurde mit Stickstoff begast. Der Ansatz wurde unter Rühren für weitere 12 h inkubiert. Nach dieser Reaktionszeit wurde der Ansatz über das Spaltensieb abgelassen und über einen Beutelfilter fein filtriert. Insgesamt wurden 9 Ansätze mit dem gleichen Enzym durchgeführt ohne eine signifikante Enzymdeaktivierung festzustellen.

Nach 4 h Reaktionszahl wiesen alle Ansätze eine Säurezahl von 25 - 30 auf, was einem Umsatz von > 85 % entspricht. Nach Beendigung der Reaktion hatten alle Ansätze eine Säurezahl von < 2, was einem Umsatz von mindestens 99 % bezogen auf die Fettsäure entspricht. Das Reaktionsprodukt war klar und annähernd farblos. Insgesamt wurden über 52.000 kg Produkt mit einer Ausbeute von über 98 % hergestellt.

### Beispiel 3: Produktqualität der nach dem erfindungsgemäßen Verfahren hergestellten Ester

Das Produkt aus Beispiel 2 wurde weiter destillativ aufgereinigt und desodoriert. Dazu wurde das Produkt über eine Produktions-Destillation bestehend aus drei hintereinander geschalteten Kolonnen gefahren. Das Produkt wurde mit einer Flussrate von 2000 kg/h dosiert und in der erste Kolonne wurde ein Vorlauf bestehend aus nicht umgesetzten Edukten mit einer Rate von 90 kg/h abgenommen. In der 2. Kolonne wurde der Ester zur farblichen Verbesserung über Kopf destilliert und in der 3. Kolonne wurde der Ester bei 120°C mit 10 % Dampf zur geruchlichen Verbesserung desodoriert.

Das so erhaltene Produkt (Mischung aus Octyloktanoat und Octyldekanoat) hatte eine Verseifungszahl von 210, eine Säurezahl von < 0,1, eine Hydroxyzahl von < 0,1 und eine Peroxidzahl von < 0,1. Die Farbzahl nach Hazen lag bei 10 und der Trübungspunkt bei - 12°C. Der Gehalt an Estern nach GC Analyse lag bei 99,4 % (Abb. 1). Aus dieser Abbildung sind die beiden Hauptpeaks Octyloktanoat (∼ 6 Min RT) und Octyldekanoat (∼ 7 Min RT) zu erkennen.

### Beispiel 4: Enzymstabilität in Abhängigkeit der Temperatur

Lewatit VPOC 1600 (Lanxess) und kommerziell erhältliche Lipase B aus *Candida antarctica* (Lipozym CalB L von Novozymes) wurden im Mengenverhältnis 1:1 über Nacht in der 20 fachen Menge Wasser bei Raumtemperatur gerührt. Das immobilisierte Enzym wurde abfiltriert und direkt zur Synthese eingesetzt.
In verschließbare Flaschen wurden jeweils 50 g einer equimolaren Mischung aus Oktanol und Fettsäure (technische Mischung aus Oktansäure und Dekansäure) mit 2 Gew% Enzymimmobilisat (1 g) zur Reaktion gebracht. Die Mischungen wurden für 22 h bei 30°C, 45°C und parallel bei 60°C geschüttelt. Nach dieser Reaktionszeit wurde der Ansatz abfiltriert und der Umsatz über Säurezahlmessung bestimmt. Insgesamt wurden 9 Ansätze mit dem gleichen Enzym bei den 3 Temperaturen durchgeführt. Die Startsäurezahl der Fettalkohol / Fettsäure Mischung lag bei 210.

| Ansatz | Säurezahl | Umsatz | Säurezahl | Umsatz | Säurezahl | Umsatz |
|---|---|---|---|---|---|---|
| | 30 °C | 30 °C | 45 °C | 45 °C | 60 °C | 60 °C |
| 1 | 30,9 | 85,3 % | 32,8 | 84,4 % | 35,3 | 83,2 % |
| 2 | 26 | 87,6 % | 40,8 | 80,6 % | 34,7 | 83,5 % |
| 3 | 24,3 | 88,4 % | 32,6 | 84,5 % | 36,2 | 82,8 % |
| 4 | 26,4 | 87,4 % | 29,4 | 86% | 36,4 | 82,7 % |
| 5 | 26,8 | 87,2% | 29,7 | 85,9 % | 91,3 | 56,5 % |
| 6 | 31,2 | 85,1 % | 31,2 | 85,1 % | 155,2 | 26,1 % |
| 7 | 25,9 | 87,7 % | 30,5 | 85,5 % | 151,8 | 27,7 % |
| 8 | 26,9 | 87,2 % | 29,7 | 85,9 % | 147,6 | 29,7 % |
| 9 | 30,8 | 85,3 % | 29,2 | 86,1 % | 173,5 | 17,4 % |

Bei den Reaktionen bei 30 °C und 45 °C ist nach 22 h Reaktionszeit über 9 Ansätze keine signifikante Abnahme der Enzymaktivität zu beobachten. Bei 60 °C ist ab dem 5. Ansatz eine deutliche Abnahme der Aktivität zu beobachten, nach 9 Ansätzen wird weniger als 25 % des anfänglichen Umsatzes erreicht.

### Beispiel 5: Enzymstabilität in Abhängigkeit der Temperatur

Accurel MP 1000 (Membrana) wurde für 1 h in einem 10fachen Überschuss an Ethanol inkubiert und dann abfiltriert. Das Ethanol-feuchte Accurel MP 1000 und kommerziell erhältliche Lipase B aus *Candida antarctica* (Lipozym CalB L von Novozymes) wurden im Mengenverhältnis 1:1 über Nacht in der 20 fachen Menge Wasser bei Raumtemperatur gerührt. Das immobilisierte Enzym wurde abfiltriert und direkt zur Synthese eingesetzt.
In verschließbare Flaschen wurden jeweils 50 g einer equimolaren Mischung aus Oktanol und Fettsäure (technische Mischung aus Oktansäure und Dekansäure) mit 2 Gew% Enzymimmobilisat (1 g) zur Reaktion gebracht. Die Mischungen wurden für 22 h bei 30 °C, 45 °C und parallel bei 60 °C geschüttelt. Nach dieser Reaktionszeit wurde der Ansatz abfiltriert und der Umsatz über Säurezahlmessung bestimmt. Insgesamt wurden 9 Ansätze mit dem gleichen Enzym bei den 3 Temperaturen durchgeführt. Die Startsäurezahl der Fettalkohol / Fettsäure Mischung lag bei 210.

| Ansatz | Säurezahl | Umsatz | Säurezahl | Umsatz | Säurezahl | Umsatz |
|---|---|---|---|---|---|---|
| | 30 °C | 30 °C | 45 °C | 45 °C | 60 °C | 60 °C |
| 1 | 27,2 | 87% | 33 | 84,3 % | 36,3 | 82,7 % |
| 2 | 25,8 | 87,8 % | 30,7 | 85,4 % | 38,2 | 81,8 % |
| 3 | 28,1 | 86,6 % | 32 | 84,8 % | 35 | 83,3 % |
| 4 | 33 | 84,3 % | 30,6 | 85,4 % | 34,2 | 83,7 % |
| 5 | 25 | 88,1 % | 30,6 | 85,4 % | 47,5 | 77,4 % |
| 6 | 24,4 | 88,4 % | 30,8 | 85,3 % | 95,6 | 54,5 % |
| 7 | 26,3 | 87,5 % | 30 | 85,7 % | 135,9 | 35,3 % |
| 8 | 26,3 | 87,5 % | 30,3 | 85,6% | 143,6 | 31,6% |
| 9 | 26,7 | 87,3 % | 30,2 | 85,6 % | 149,4 | 28,9 % |

Bei den Reaktionen bei 30 °C und 45 °C ist nach 22 h Reaktionszeit über 9 Ansätze keine signifikante Abnahme der Enzymaktivität zu beobachten. Bei 60 °C ist ab dem 5. Ansatz eine leichte und ab dem 6. Ansatz eine deutliche Abnahme der Aktivität zu beobachten. Nach 9 Ansätzen wird nur noch etwa 35 % des anfänglichen Umsatzes erreicht.

Ein weiterer Ansatz wurde bei 60 °C und einem Vakuum von 20 mbar in einem Doppelmantelreaktor durchgeführt. Pro Ansatz wurden 800 g der oben beschriebenen Edukt-Mischung eingesetzt und 2 % auf Accurel MP 1000 immobilisierte Lipase B aus *Candida antarctica* eingesetzt. Insgesamt wurden 8 Ansätze unter kontinuierleichem Rühren und Stickstoffbegasung durchgeführt. Nach 3 h und 22 h wurden jeweils Proben entnommen. Eine Akkumulation von Wasser wurde nicht beobachtet.

| Ansatz | Säurezahl 3 h | Umsatz 3 h | Säurezahl 22 h | Umsatz 22 h |
|---|---|---|---|---|
| 1 | 8,9 | 95,8 % | 3,1 | 98,5 % |
| 2 | 8,5 | 95,9 % | 5,1 | 97,6 % |
| 3 | 9,9 | 95,3 % | 0,4 | 99,8 % |
| 4 | 25,0 | 88,1 % | 0,4 | 99,8 % |
| 5 | 16,6 | 92,1 % | 0,6 | 99,7 % |
| 6 | 91,9 | 56,2 % | 5,2 | 97,5 % |
| 7 | 169,6 | 19,2 % | 40,5 | 80,7 % |
| 8 | 170,2 | 19% | 66,3 | 68,4 % |

Auch unter Vakuumbedingungen und kontinuierlicher Entfernung von Wasser tritt bei der Reaktionstemparatur von 60 °C eine schnelle Deaktivierung des Katalysators ein. Nach 4 Zyklen ist bei der Probennahme nach 3 h bereits eine Verlangsamung der Reaktionsgeschwindigkeit zu erkennen, nach 6 - 7 Zyklen wird auch nach 22 h kein Vollumsatz mehr erreicht.

Die Beispiele 1, 4 und 5 wurden mit vergleichbaren Enzymmengen und mit der gleichen Mischung an Edukten durchgeführt. Bei der 3-stufigen Fahrweise der Estersynthese (Beispiel 1) mit einer Reaktion ins Gleichgewicht bei 45 °C und einer weiteren Umsetzung unter Vakuum bei 60 °C ist über 10 Reaktionszyklen keine signifikante Abnahme der Enzymaktivität zu erkennen. Dagegen führte die direkte Reaktion bei 60 °C sowohl mit dem Träger Lewatit VPOC 1600 (Beispiel 4) und Accurel MP 1000 (Beispiel 5) bereits nach 5 Zyklen zu einer signifikanten Reduktion der Enzymaktivität.

Aus dem Vergleich zeigt sich, dass insbesondere die in den Beispielen verwendeten Edukte einen deaktivierenden Einfluss auf das Enzym ausüben. Oktanol hat einen starken Lösungsmittelcharakter und Oktan- sowie Dekansäure sind noch recht starke Säuren. Die Deaktivierung des Enzyms ist dabei stark von der gewählten Reaktionstemperatur abhängig. Über die dreistufige Synthese, bei der im ersten Schritt > 80 % der Edukte bei niedriger Temperatur und dann die restlichen 10 - 20 % der Edukte bei einer höheren Temperatur umgesetzt werden, führt zu einer deutlich verbesserten Enzymstabilität im Vergleich zur direkten Umsetzung der Edukte bei hoher Temperatur. Die dreistufige Fahrweise ist insbesondere für die technische Umsetzung geeignet, da die Wasserentfernung unter Vakuum in der zweiten Reaktionsstufe deutlich besser funktioniert als bei niedriger Temperatur.

In weiteren Lagertests wurde bestätigt, dass das Enzym im produzierten Ester eine bessere Stabilität hat als in der Edukt-Mischung. Die Lagerung des Immobilisates für 1 Monat bei 45°C in der Produktmischung führte zu keiner signifikanten Abnahme der Enzymaktivität.

### Vergleichsbeispiel 1: Estersynthese unter Vakuum in einem 10 m³ Reaktor

Die Immobilisation und Reaktion wurde im gleichen Reaktor wie in Beispiel 2 beschrieben durchgeführt. Die Immobilisation wurde ebenfalls wie in Beispiel 2 beschrieben durchgeführt und die Eduktmischung sowie das Verhältnis aus Biokatalysator und Edukten waren identisch.
In den Reaktor wurden zum gesamten Enzymimmobilisat 6550 kg eine Mischung aus Oktanol und Fettsäure (technische Mischung aus Oktansäure und Dekansäure) gegeben. Die Mischung wurde für 4 h bei 45 °C und Stickstoffbegasung bei einem Strahler-Vakuum von 30 mbar gerührt. Nach 4 h wurde eine Probe genommen und die Menge an vorhandenem Wasser wurde analysiert. Die Säurezahl der Probe lag bei 26, was einem Umsatz von > 87 % entspricht.
Es zeigte sich, dass sich nach 4 h Wasser im Reaktor akkumuliert: etwa 75 % der gebildeten Wassermenge konnte bei der Temperatur von 45°C nicht durch die Vakuumpumpen aus dem Reaktor entfernt werden. Zur Entfernung des anfänglich gebildeten Wassers durch Verdampfung wären bei der gegebenen Temperatur etwa 16 h notwendig gewesen. Erst nach vollständiger Entfernung der Wasserphasen hätte das System weiter reagieren können.

Es hat sich gezeigt, dass in typischen Produktionsreaktoren für kosmetische und technische Ester, die eine Größe von 20 - 100 m³ aufweisen, die Wasserentfernung aufgrund von höherem Füllstand und ungünstigerer Oberfläche zu Volumen Verhältnis länger dauert. Nur durch Erhöhung der Reaktionstemperatur auf z.B. 80 - 100 °C kann das Wasser schneller entfernt werden. Da hier aber die Deaktivierung des Enzyms sehr stark ist, ist diese Verfahrensvariante keine geeignete Lösung.

Wie im erfindungsgemäßen Beispiel 2 gezeigt wurde, wird die Reaktion nach 4 h gestoppt, so dass die Wasserphase innerhalb kurzer Zeit aus dem Reaktor entfernt werden kann. Auch bei niedriger Temperatur (30 - 45 °C) ist die Reaktion bis in das Gleichgewicht innerhalb von etwa 4 h erreicht. Nach der Abtrennung des Wassers kann die Reaktionstemperatur zur besseren Abtrennung der Wasserphase auf z.B. 60 °C ohne eine signifikante Enzymdeaktivierung angehoben werden, da bereits der Großteil der Edukte umgesetzt wurde. Dadurch kann die Gesamtreaktion innerhalb von 12 - 20 h durchgeführt werden.

## Patentansprüche

1. Verfahren zur Herstellung von Estern aus Fettalkoholen und Fettsäuren, das die Schritte umfasst:
- Umsetzen von Fettalkoholen der Formel R¹-OH, worin R¹ eine aliphatische lineare oder verzweigte Kohlenwasserstoffgruppe mit 6 bis 22 Kohlenstoffatomen bedeutet, und Fettsäuren der Formel R²-COOH, worin R² eine aliphatische lineare oder verzweigte Kohlenwasserstoffgruppe mit 6 bis 22 Kohlenstoffatomen bedeutet, in Gegenwart von Lipase bei einer Temperatur im Bereich von 30 bis 50 °C, bis das Reaktionsgleichgewicht erreicht ist;
- Entfernen des bei der Umsetzung gebildeten Wassers und
- Vervollständigen der Umsetzung unter Vakuum bei einer Temperatur von 50 bis 80 °C.

2. Verfahren nach Anspruch 1, **dadurch gekennzeichnet, dass** die Vervollständigung der Umsetzung in einer Stripgasatmosphäre durchgeführt wird.

3. Verfahren nach Anspruch 2, **dadurch gekennzeichnet, dass** als Stripgas Stickstoff, Luft, Kohlendioxid oder Argon verwendet wird.

4. Verfahren nach einem der Ansprüche 1 bis 3, **dadurch gekennzeichnet, dass** bei der Vervollständigung der Umsetzung ein Druck von 100 bis 10.000 Pa angewendet wird.

5. Verfahren nach Anspruch 4, **dadurch gekennzeichnet, dass** bei der Vervollständigung der Umsetzung ein Druck von 1.500 bis 4.500 Pa angewendet wird.

6. Verfahren nach einem der Ansprüche 1 bis 5, **dadurch gekennzeichnet, dass** die Umsetzung bei einer Temperatur im Bereich von 35 bis 45 °C durchgeführt wird.

7. Verfahren nach einem der Ansprüche 1 bis 6, **dadurch gekennzeichnet, dass** die Vervollständigung der Umsetzung bei einer Temperatur im Bereich von 55 bis 65 °C durchgeführt wird.

8. Verfahren nach einem der Ansprüche 1 bis 7, **dadurch gekennzeichnet, dass** die Lipase immobilisiert verwendet wird.

9. Verfahren nach einem der Ansprüche 1 bis 8, **dadurch gekennzeichnet, dass** die immobilisierte Lipase in einer Menge von 0,5 bis 5,0 Gew.-% eingesetzt wird.

10. Verfahren nach einem der Ansprüche 1 bis 9, **dadurch gekennzeichnet, dass** als Fettalkohole solche eingesetzt werden, deren lineare oder verzweigte Alkyl- oder Alkenylgruppe 8 bis 18 Kohlenstoffatome aufweist.

11. Verfahren nach einem der Ansprüche 1 bis 10, **dadurch gekennzeichnet, dass** als Fettsäuren solche eingesetzt werden, deren lineare oder verzweigte Alkyl- oder Alkenylgruppe 8 bis 18 Kohlenstoffatome aufweist.

12. Verfahren nach einem der Ansprüche 1 bis 11, **dadurch gekennzeichnet, dass** die Esterherstellung im Eintopfverfahren erfolgt.

13. Verfahren nach einem der vorangegangenen Ansprüche, **dadurch gekennzeichnet, dass** weiterhin Aufreinigungs- und Konservierungsschritte durchgeführt werden.

14. Verfahren nach Anspruch 13, **dadurch gekennzeichnet, dass** eine Vorlaufdestillation, eine Produktdestillation zur farblichen Verbesserung, eine Desodorierung zur Geruchsoptimierung und/oder eine Zugabe von Antioxidantien durchgeführt werden.

## Claims

1. A process for the production of esters of fatty alcohols and fatty acids that comprises the steps:
- reacting fatty alcohols of the formula R¹-OH, where R¹ is an aliphatic linear or branched hydrocarbon group having 6 to 22 carbon atoms, and fatty acids of the formula R²-COOH, where R² is an aliphatic linear or branched hydrocarbon group having 6 to 22 carbon atoms, in the presence of lipase at a temperature in the range from 30 to 50°C, until the reaction equilibrium is achieved;
- removing the water formed during the reaction and
- completing the reaction under vacuum at a temperature from 50 to 80°C.

2. The process according to claim 1, wherein the completion of the reaction is carried out in a stripping gas atmosphere.

3. The process according to claim 2, wherein the stripping gas used is nitrogen, air, carbon dioxide or argon.

4. The process according to any one of claims 1 to 3, wherein a pressure from 100 to 10,000 Pa is employed during the completion of the reaction.

5. The process according to claim 4, wherein a pressure from 1,500 to 4,500 Pa is employed during the completion of the reaction.

6. The process according to any one of claims 1 to 5, wherein the reaction is carried out at a temperature in the range from 35 to 45°C.

7. The process according to any one of claims 1 to 6, wherein the completion of the reaction is carried out at a temperature in the range from 55 to 65°C.

8. The process according to any one of claims 1 to 7, wherein the lipase is used in immobilized form.

9. The process according to any one of claims 1 to 8, wherein the immobilized lipase is used in an amount from 0.5 to 5.0 weight%.

10. The process according to any one of claims 1 to 9, wherein the fatty alcohols used are those the linear or branched alkyl or alkenyl group of which has 8 to 18 carbon atoms.

11. The process according to any one of claims 1 to 10, wherein the fatty acids used are those the linear or branched alkyl or alkenyl group of which has 8 to 18 carbon atoms.

12. The process according to any one of claims 1 to 11, wherein the ester production proceeds in the one-pot process.

13. The process according to any one of the preceding claims, wherein purification and preservation steps are additionally carried out.

14. The process according to claim 13, wherein a forerun distillation, a product distillation for color improvement, a deodorization for odor optimization and/or an addition of antioxidants are carried out.

## Revendications

1. Procédé de préparation d'esters à partir d'alcools gras et d'acides gras, qui comprend les étapes :
- réaction d'alcools gras de formule R¹-OH, dans laquelle R¹ est un groupe hydrocarboné aliphatique à chaîne droite ou ramifiée ayant 6 à 22 atomes de carbone, et d'acides gras de formule R²-COOH, dans laquelle R² est un groupe hydrocarboné aliphatique à chaîne droite ou ramifiée ayant 6 à 22 atomes de carbone, en présence de lipase à une température comprise dans la plage de 30 à 50°C, jusqu'à atteindre l'équilibre de réaction ;
- élimination de l'eau formée lors de la réaction, et
- parachèvement de la réaction sous vide à une température de 50 à 80°C.

2. Procédé selon la revendication 1, **caractérisé en ce que** le parachèvement de la réaction est mis en oeuvre dans une atmosphère de gaz de strippage.

3. Procédé selon la revendication 2, **caractérisé en ce qu'**on utilise en tant que gaz de strippage l'azote, l'air, le dioxyde de carbone ou l'argon.

4. Procédé selon l'une des revendications 1 à 3, **caractérisé en ce que**, lors du parachèvement de la réaction, on utilise une pression de 100 à 10 000 Pa.

5. Procédé selon la revendication 4, **caractérisé en ce que**, lors du parachèvement de la réaction, on utilise une pression de 1500 à 4500 Pa.

6. Procédé selon l'une des revendications 1 à 5, **caractérisé en ce que** la réaction est mise en oeuvre à une température comprise dans la plage de 35 à 45°C.

7. Procédé selon l'une des revendications 1 à 6, **caractérisé en ce que** le parachèvement de la réaction est mis en oeuvre à une température comprise dans la plage de 55 à 65°C.

8. Procédé selon l'une des revendications 1 à 7, **caractérisé en ce que** la lipase est utilisée immobilisée.

9. Procédé selon l'une des revendications 1 à 8, **caractérisé en ce que** la lipase immobilisée est utilisée en une quantité de 0,5 à 5,0 % en poids.

10. Procédé selon l'une des revendications 1 à 9, **caractérisé en ce qu'**on utilise en tant qu'alcools gras ceux dont le groupe alkyle ou alcényle à chaîne droite ou ramifiée a 8 à 18 atomes de carbone.

11. Procédé selon l'une des revendications 1 à 10, **caractérisé en ce qu'**on utilise en tant qu'acides gras ceux dont le groupe alkyle ou alcényle à chaîne droite ou ramifiée a 8 à 18 atomes de carbone.

12. Procédé selon l'une des revendications 1 à 11, **caractérisé en ce que** la préparation des esters est réalisée par un procédé monotope.

13. Procédé selon l'une des revendications précédentes, **caractérisé en ce qu'**on met en oeuvre en outre des étapes de purification et de conservation.

14. Procédé selon la revendication 13, **caractérisé en ce qu'**on met en oeuvre une première distillation, une distillation du produit pour améliorer la couleur, une désodorisation pour optimiser l'odeur et/ou une addition d'antioxydants.
